# EUROPEAN PATENT APPLICATION

(11) **EP 4 450 063 A1**
(43) Date of publication of application: **23.10.2024**
(21) Application number: 23460011.2
(22) Date of filing: 21.04.2023
(51) Int. Cl.: A61K 9/00, A61K 33/242, A61K 33/243, A61K 33/34, A61K 33/38

(54) **BREATHING MIXTURE AND ITS USE**

(71) Applicant: Daremiak, Lukasz, 00-544 Warszawa (PL)
(72) Inventor: Daremiak, Lukasz, 00-544 Warszawa (PL)
(74) Representative: Pomianek, Grazyna

(57) **Abstract**

The breathing mixture containing air is characterised in that it comprises oxygen in the volumetric proportion of 43 % to 64 %, carbon dioxide in the volumetric proportion of 2.4 % to 3.6%, hydrogen in the volumetric proportion of 0.4% to 0.6%, and nitrogen alongside other air components in the volumetric proportion of no more than 54.2 %. In addition, the breathing mixture may comprise nanoparticles of: gold in the volumetric proportion of 0.00004% to 0.00006% and/or silver in the volumetric proportion of 0.00008% to 0.00012% and/or copper in the volumetric proportion of 0.000008% to 0,000012% and/or platinum in the volumetric proportion of 0.000008% to 0.000012%, where in each case the amounts of all components of the breathing mixture add up to 100% of the breathing mixture volume. The breathing mixture is under the pressure of 1200 hPa to 2000 hPa, and the temperature of the breathing mixture is 20 °C to 22 °C.

## Description

The subject matter of the invention is a breathing mixture and its use. In particular, the invention concerns the composition of the breathing mixture and its use.

The air we breathe is a mixture of gases found in the lowest part of the Earth's atmosphere. This composition has varied over the earth's history over a very wide range. The concentrations of the main air constituents have varied. Nowadays, the content of the main air constituents varies very little. At present, the volumetric composition of pure dry air is as follows: 78.06% nitrogen; 20.98% oxygen; 0.93% argon; 0.03% other.

From patent document PL 430 025 A1, a normobaric system forming a chamber for subterraneotherapy mounted inside an underground gallery is known. The system comprises a normobaric chamber filled with compressed air coming from a cave connected to the gallery by an air conditioning system and a compressor. The cave air has site-specific composition and physical properties. The aim of the invention is to use natural healing factors found in nature, including a low CO₂ concentration and a pH of no more than 5.5.

From patent document US 2018/0264213 A1, a gas mixture is known used to ventilate passengers and flight crew in emergency situations. Depending on altitude, the mixture contains 7 ± 5% CO₂ at a flight altitude of 15,000 feet, increasing to 17 ± 5% CO₂ at a flight altitude of 30000 ft. Carbon dioxide improves the bioavailability of oxygen in the body. The gas mixture is produced by additionally dosing CO₂ either to pure O₂ , or to a gas mixture containing an N₂ fraction and an O₂ fraction. The method involves making the above gas mixture available through masks respiratory.

Hyperbaric chambers are also known to be medical devices, either single or multi-person. Hyperbaric therapy is a therapeutic treatment based on the use of pure oxygen under conditions of sufficiently high pressure. Hyperbaric oxygen therapy is used to treat a wide range of conditions, under medical supervision. Treatments in a hyperbaric chamber last no longer than 45-60 minutes.

The purpose of the invention is to develop the composition of the breathing mixture intended for the group of healthy people and those suffering from various medical conditions, used in a chamber where one can stay and function with no time limits to relax and regenerate the organism. In addition, the invention solves e.g. the problem of overcoming the physiological barriers of oxygen penetration to the cells of the organism.

According to the present invention, a breathing mixture containing air is characterised in that it comprises oxygen in the volumetric proportion of 43 % to 64 %, carbon dioxide in the volumetric proportion of 2.4 % to 3.6%, hydrogen in the volumetric proportion of 0.4% to 0.6%, and nitrogen alongside other air components in the volumetric proportion of no more than 54.2 %, where the breathing mixture is under the pressure between 1200 hPa and 2000 hPa, and the temperature of the breathing mixture is 20 °C to 22 °C.
The term 'other air components' covers air components other than nitrogen, oxygen, carbon dioxide, and hydrogen.
Preferably, a breathing mixture described above further comprises: nanoparticles of gold in the volumetric proportion of 0.00004% to 0.00006%, and/or nanoparticles of silver in the volumetric proportion of 0.00008% to 0.00012%, and/or nanoparticles of copper in the volumetric proportion of 0.000008% to 0.000012%, and/or nanoparticles of platinum in the volumetric proportion of 0.000008% to 0.000012%, where in each case the amounts of all components of the breathing mixture add up to 100% of the breathing mixture volume.
Most preferably, a breathing mixture contains oxygen in the volumetric proportion of 54%, carbon dioxide in the volumetric proportion of 3%, hydrogen in the volumetric proportion of 0.5%, nanoparticles of gold in the volumetric proportion of 0.00005%, nanoparticles of silver in the volumetric proportion of 0.0001%, nanoparticles of copper in the volumetric proportion of 0.00001%, nanoparticles of platinum in the volumetric proportion of 0.00001%, and nitrogen alongside other air components in the volumetric proportion of 42.49983 %.
A breathing mixture described above is used in relaxation therapy and regeneration therapy.

A breathing mixture according to the present invention is intended for use in closed rooms made of metal, concrete, epoxide, textile, all withstanding pressure up to 2000 hPa, resistant to deformation, and preventing loss of hydrogen from the room.
The breathing mixture is produced by dosing oxygen, carbon dioxide, hydrogen, and additionally nanoparticles of silver, and/or gold, and/or copper, and/or platinum into the existing air under increased pressure, in a tightly closed room (chamber).
Rooms with the breathing mixture according to the invention enable staying and functioning there with no time limits.

Increased atmospheric pressure along with increased volumes of oxygen, carbon dioxide, hydrogen and nanoparticles of gold and/or silver and/or copper and/or platinum produce a range of health-promoting and regenerative effects. The increased pressure overcomes physiological barriers to oxygen penetration into the cells and allows increased doses of oxygen to be delivered to the cells, even to the non-vascularised areas of the body.

Under normal conditions, the supply of oxygen to the body remains dependent on the blood supply to a specific area of the body. With the conditions according to the invention, oxygen has the property of penetrating even into non-vascularised areas of the body.

The use of an increased (almost 100-fold) carbon dioxide content in the mixture according to the invention is justified by the scientific work of Christian Bhor, who as early as 1904 stated that in the case of a high carbon dioxide content, the affinity of haemoglobin for oxygen is reduced, which in turn causes it to release oxygen more easily into the cells. This phenomenon, which occurs in the body, was named the 'Bhor effect'. This effect, together with the pressure and amount of oxygen, has a much better effect than hyperbaria.

Numerous scientific studies have shown that hydrogen is the best antioxidant fighting against free oxygen radicals formed in our body during various biochemical processes. A deficiency of antioxidants in the body and an increase in oxygen free radicals results in so- called oxidative stress and the consequent development of a number of diseases, including heart disease, brain disease, diabetes, pancreatic and liver disease and various types of cancer. Molecular hydrogen inhalation has already begun to be used in Japan and Korea, however, the solution according to the invention is much more effective as, together with increased pressure and the Bhor Effect, the hydrogen is able to reach anywhere in the body and neutralise inflammation.

Enrichment of the mixture with gold, silver, platinum and copper nanoparticles with a monocrystalline non-ionic structure results in easier absorption and prolonged action.

It is known that:
- gold has strong collagen synthesis-stimulating properties and is therefore widely used in cosmetics (creams with gold flakes, 'gold threads');
- silver has strong antibacterial and anti-inflammatory properties;
- platinum acts as a stimulant and regulator in enzymatic, respiratory and circulatory processes;
- copper is essential for the absorption and metabolism of iron and supports the oxidation of vitamin C. In addition, through the synthesis of dopamine, it influences the development of the nervous system, and through the synthesis of collagen and elastin, the regeneration of connective tissue.

It is known that respiration produces molecules that are one of the causes of skin ageing. The body is able to neutralise them by producing antioxidants, e.g. glutathione and melatonin, but as a result of too many factors acting at once, such as environmental pollution, poor diet (poor in nutrients, vitamins and trace elements), poor lifestyle, UV radiation, the number of free radicals increases rapidly and so-called oxidative stress occurs. The purpose of enriching the mixture with additional hydrogen is so that the hydrogen naturally absorbed by the body can neutralise the free radicals (this reaction results in water that is harmless to our body). As a result, the skin is not prone to wrinkles, ageing processes occur much more slowly and collagen and elastin fibres become stronger. In addition, these processes are supported by nanoparticles of the gold and copper elements. As a result, one can count on a visible rejuvenating effect, reduction of cellulite, correction of scars and biological renewal.

Furthermore, it is believed that the mixture according to the invention may also have applications in conditions such as: Alzheimer's; asthma; autism and Asperger's syndrome; atopic dermatitis; Lyme disease; diabetes; autoimmune diseases; depression; endometriosis; fas; Hashimoto's; hypoxia; pain management; fertility treatment; psoriasis; metabolism; migraine; natural doping; cancer; aesthetic medicine; cerebral palsy; preparation for pregnancy; burns; obesity and overweight; Parkinson's disease; recovery after surgery; recovery after Covid; rehabilitation; rheumatism; multiple sclerosis; stroke; concussion; strengthening the body before planned surgical procedures; heart attack; chronic fatigue syndrome.

The invention is described in further detail below, in its embodiments. The breathing mixture is obtained as follows: the pressure of the air in a tightly closed room is increased with a compressor, then the content of medical oxygen is increased up to the pre-set level using an oxygen concentrator; the content of medical hydrogen is increased to the pre-set level with a medical hydrogen generator; the content of medical carbon dioxide is increased to the pre-set level using a device; nanoparticles of gold and/or silver and/or copper and/or platinum in their pre-set volumes are sprayed with a diffusor. The said devices areelectronically controlled with a specialist software so as to achieve the pre-set composition of the breathing mixture, its pressure, and temperature.

### Embodiment I

The breathing mixture contains:
- oxygen in the volumetric proportion of 54%
- carbon dioxide in the volumetric proportion of 3%
- hydrogen in the volumetric proportion of 0.5%
- nanoparticles of gold in the volumetric proportion of 0.00005%
- nanoparticles of silver in the volumetric proportion of 0.0001%
- nanoparticles of copper in the volumetric proportion of 0.00001%
- nanoparticles of platinum in the volumetric proportion of 0.00001%
- nitrogen and other air components in the volumetric proportion of 42.49983 %,
where the breathing mixture is under the pressure of 1500 hPa, and the temperature of the breathing mixture is 21°C.

### Embodiment II

The breathing mixture contains:
- oxygen in the volumetric proportion of 43%
- carbon dioxide in the volumetric proportion of 3.6%
- hydrogen in the volumetric proportion of 0.4%
- nanoparticles of gold in the volumetric proportion of 0.00004%
- nanoparticles of silver in the volumetric proportion of 0.00008%
- nanoparticles of copper in the volumetric proportion of 0.000008%
- nanoparticles of platinum in the volumetric proportion of 0.000008%
- nitrogen and other air components in the volumetric proportion of 52.999864 %,
where the breathing mixture is under the pressure of 1500 hPa, and the temperature of the breathing mixture is 21°C.

### Embodiment III

The breathing mixture contains:
- oxygen in the volumetric proportion of 64%
- carbon dioxide in the volumetric proportion of 2.4%
- hydrogen in the volumetric proportion of 0.6%
- nanoparticles of gold in the volumetric proportion of 0.00006%
- nanoparticles of silver in the volumetric proportion of 0.00012%
- nanoparticles of copper in the volumetric proportion of 0.000012%
- nanoparticles of platinum in the volumetric proportion of 0.000012%
- nitrogen and other air components in the volumetric proportion of 32.999796 %,
where the breathing mixture is under the pressure of 1500 hPa, and the temperature of the breathing mixture is 21°C.

### Embodiment IV

The breathing mixture contains:
- oxygen in the volumetric proportion of 64%
- carbon dioxide in the volumetric proportion of 3.6%
- hydrogen in the volumetric proportion of 0.6%
- nitrogen and other air components in the volumetric proportion of 31.8 %, where the breathing mixture is under the pressure of 1500 hPa, and the temperature of the breathing mixture is 21°C.

### Embodiment V

The breathing mixture contains:
- oxygen in the volumetric proportion of 43%
- carbon dioxide in the volumetric proportion of 2.4%
- hydrogen in the volumetric proportion of 0.4%
- nitrogen and other air components in the volumetric proportion of 54.2 %, where the breathing mixture is under the pressure of 1500 hPa, and the temperature of the breathing mixture is 21°C.

In other embodiments, the breathing mixture containing oxygen, carbon dioxide, and hydrogen in the volumes as in embodiments I to III may contain nanoparticles of gold or silver or copper or platinum in the volumes as in embodiments I to III, respectively, where in each case the remaining component is nitrogen plus other air components (i.e. in each case the volumes of all components of the breathing mixture add up to 100% of the breathing mixture volume).

In all embodiments, the breathing mixture may be under the pressure of 1200 hPa to 2000 hPa, and the temperature of the mixture may be 20 °C to 22 °C.

Therapy sessions can last from two hours to a full day's stay and require no prior preparation. An experiences show that the first effects are noticeable after the first hour of stay. The best effects are obtained during night stays, when we fall asleep and the body regenerates. No side effects have been reported.

Before the start of each session, footwear must be changed and outerwear left in the dressing room. Then, you proceed to the airlock, where compression is carried out, i.e. the atmospheric pressure is equalised to that prevailing in the chamber. Once the compression process in the airlock is complete, one enters the main chamber room, where one spends the rest of the session. After the therapy, one goes back into the airlock for decompression, which allows one to leave the chamber.

Compression is the process of equalising the pressure prevailing outside i.e. ~about 1000 hPa to the pressure prevailing in the chamber i.e. about 1500 hPa. Decompression, on the other hand, is the reverse process, i.e. equalising the pressure to that currently prevailing in the environment.

The compression process takes about 7 minutes and decompression about minutes.
Anyone can enter the chamber, regardless of age. They can be very young children, adults as well as the elderly.

Oxygen chamber therapy can be compared to exercise in a gym. If you visit once a month, you will not feel any effects. It is therefore recommended to have a session every day for 2 to 12 hours.

## Claims

1. A breathing mixture containing air, **characterised in that** it comprises oxygen in the volumetric proportion of 43 % to 64 %, carbon dioxide in the volumetric proportion of 2.4 % to 3.6%, hydrogen in the volumetric proportion of 0.4% to 0.6%, and nitrogen alongside other air components in the volumetric proportion of no more than 54.2 %, where the breathing mixture is under the pressure between 1200 hPa and 2000 hPa, and the temperature of the breathing mixture is 20 °C to 22 °C.

2. The mixture according to claim 1, **characterised in that** it further comprises nanoparticles of gold in the volumetric proportion of 0.00004% to 0.00006%, and/or nanoparticles of silver in the volumetric proportion of 0.00008% to 0.00012%, and/or nanoparticles of copper in the volumetric proportion of 0.000008% to 0.000012%, and/or nanoparticles of platinum in the volumetric proportion of 0.000008% to 0.000012%, where in each case the amounts of all components of the breathing mixture add up to 100% of the breathing mixture volume.

3. The mixture according to claim 2, **characterised in that** it comprises oxygen in the volumetric proportion of 54%, carbon dioxide in the volumetric proportion of 3%, hydrogen in the volumetric proportion of 0.5%, nanoparticles of gold in the volumetric proportion of 0.00005%, nanoparticles of silver in the volumetric proportion of 0.0001%, nanoparticles of copper in the volumetric proportion of 0.00001%, nanoparticles of platinum in the volumetric proportion of 0.00001%, and nitrogen alongside other air components in the volumetric proportion of 42.49983 % where the breathing mixture is under the pressure between 1500 hPa, and the temperature of the breathing mixture is 20 °C to 22 °C.

4. The use of a breathing mixture according to any one of the claims 1 to 3 in the relaxation therapy.

5. The use of a breathing mixture according to any one of the claims 1 to 3 in regenerative therapy.
